# EUROPÄISCHE PATENTANMELDUNG

(11) **EP 3 748 359 A1**
(43) Veröffentlichungstag der Anmeldung: **09.12.2020**
(21) Anmeldenummer: 20171507.5
(22) Anmeldetag: 17.08.2016
(51) Int. Cl.: G01N 33/537

(54) **BAKTERIEN-SCHNELLTEST**

(30) Priorität: 17.08.2015 EP 15181240
(62) Teilanmeldung aus: 16753650.7
(71) Anmelder: Swiss Analyze Bacteria AG, 8274 Tägerwilen (CH)
(72) Erfinder: Ebert, Dieter, 8274 Gottlieben (CH); Reichl, Mathias, 93309 Kehlheim (DE); Lubitz, Werner, 3420 Kritzendorf (AT)
(74) Vertreter: Weiss, Wolfgang

(57) **Zusammenfassung**

Die Erfindung betrifft Verfahren und Vorrichtungen zur quantitativen Bestimmung von Bakterien.

## Beschreibung

Die Erfindung betrifft Verfahren und Vorrichtungen zur quantitativen Bestimmung von Bakterien.

Legionellen sind eine Gattung gramnegativer Bakterien. Optimale Bedingungen für ihre Vermehrung finden sie in Wasser in einem Temperaturbereich von 25-50 °C. Sie können in Anlagen für die Warmwassererzeugung und -verteilung, in Schwimmbädern, Luftwäschern für Klimaanlagen, Kühltürmen, Wassertanks und Wasserleitungen mit langen Stillstandszeiten vorkommen. Das Einatmen von legionellenhaltigem Wasser als Aerosol, z.B. beim Duschen, bei Klimaanlagen, durch Rasensprenger oder in Whirlpools, kann beim Menschen zu einer Infektion führen, die eine gefährliche, in vielen Fällen tödlich verlaufende Krankheit, die Legionellose, hervorrufen kann.

Die deutsche Trinkwasserverordnung schreibt daher eine regelmäßige Untersuchungspflicht auf Legionellen vor, die Inhaber von Trinkwasserinstallationen mit Großanlagen zur Trinkwassererwärmung betrifft, sofern aus diesen Wasser abgegeben wird und es dabei zu dessen Vernebelung, z.B. in Duschen, kommt. Diese Vorschrift gilt unter anderem für Krankenhäuser, Schulen, Kindergärten, Hotels und Pflegeheime. Diese Einrichtungen sind verpflichtet, einmal jährlich eine Untersuchung auf Legionellen durchzuführen. Seit neuerem müssen auch Eigentümer bzw. Vermieter von Mehrfamilienhäusern, Wohnungsbaugesellschaften und Hausverwaltungen entsprechende Untersuchungen in einem Intervall von jeweils drei Jahren durchführen.

In der Trinkwasserverordnung ist für Legionellen ein technischer Maßnahmenwert von 100 koloniebildenden Einheiten (KBE) je 100 ml festgelegt. Wird bei einer Untersuchung eine Überschreitung dieses Wertes festgestellt, muss unmittelbar eine Meldung an das zuständige Gesundheitsamt erfolgen.

Die Analytik im Rahmen der Untersuchungspflicht erfolgt mittels klassischer mikrobiologischer Nachweisverfahren. Die zu untersuchenden Trinkwasserproben werden im Labor aufgeteilt und in zwei parallelen Ansätzen gemäß ISO 11731 (1998) und DIN EN ISO 11731-2 (2008) untersucht. Ein Teil der Ausgangsprobe wird in einem Direktansatz und ein anderer Teil nach Filtration in Petrischalen mit Agar-Medium aufgebracht. Die Agar-Platten aus beiden Ansätzen werden zehn Tage bei einer konstanten Temperatur von 36 ± 2 °C im Brutschrank inkubiert. Danach werden während der Bebrütung gewachsene Kolonien von Legionellen gezählt und ausgewertet. Das quantitative Ergebnis wird in *"koloniebildenden Einheiten"* (KBE) bezogen auf 100 ml Probe angegeben.

Zur Bestätigung charakteristischer Legionellen-Kolonien werden mindestens fünf Kolonien parallel sowohl auf Cystein-haltigem Medium als auch auf einem Cystein-freien Medium mindestens zwei Tage lang subkultiviert. Da die Aminosäure Cystein für Legionellen essentiell ist, gilt der Nachweis als bestätigt, wenn die Kolonie auf dem Cystein-haltigen Medium wächst, nicht jedoch auf dem Cystein-freien Medium.

Ein Nachteil der oben genannten Analytik besteht darin, dass das mikrobiologische Nachweisverfahren kostspielig und zeitaufwändig ist. Ein weiterer Nachteil des Verfahrens besteht darin, dass die damit erhaltenen Ergebnisse eine Ungenauigkeit von zwischen ca. 15 und 35 % aufweisen.

Die der vorliegenden Erfindung zugrunde liegende Aufgabe bestand somit darin, ein verbessertes Verfahren zur quantitativen Bestimmung von Legionellen und auch anderen Bakterien bereitzustellen, bei dem die Nachteile des Standes der Technik zumindest teilweise vermieden werden können. Insbesondere soll das Verfahren eine erheblich schnellere Bestimmung mit hoher Genauigkeit erlauben.

Zur Lösung dieser Aufgabe werden neue Verfahren und Vorrichtungen zum quantitativen Nachweis von Bakterien bereitgestellt. Die Erfindung beruht auf einer Messung der markierten Bakterien, wobei das erhaltene Messsignal mit der Gesamtzahl der in der Probe vorhandenen Bakterien korreliert wird. Der Nachweis der Bakterien kann beispielsweise durch Lumineszenz-, Fluoreszenz- oder Raman-Strahlung erfolgen.

Ein erster Aspekt der Erfindung betrifft ein Verfahren zur quantitativen Bestimmung von Bakterien in einer Probe, umfassend die Schritte:
(a) Inkubieren der zu untersuchenden Probe mit einem an die zu bestimmenden Bakterien bindefähigen Markierungsreagenz, vorzugsweise ausgewählt aus einem fluoreszierenden Markierungsreagenz, einem lumineszierenden Markierungsreagenz und einem Raman-Markierungsreagenz,
(b) Entfernen von nicht an die Bakterien gebundenem Markierungsreagenz von den Bakterien durch eine für die zu bestimmenden Bakterien undurchlässige und für das Markierungsreagenz durchlässige Membran, wobei die zu bestimmenden Bakterien auf der Membran gesammelt werden,
(c) Bestimmen des an die gesammelten Bakterien gebundenen Markierungsreagenz vorzugsweise durch Lumineszenz-, Fluoreszenz- und/oder Ramanmessung und
(d) quantitatives Auswerten des Messsignals aus Schritt (c).

Das erfindungsgemäße Verfahren erlaubt eine schnelle und quantitative Bestimmung von Bakterien in einer Probe. Es kann direkt nach Probennahme und ohne vorherige Kultivierung der zu bestimmenden Bakterien durchgeführt werden. Die Inkubationszeit, die für die Bindung des Markierungsreagenz an die Bakterien erforderlich ist, liegt vorzugsweise bei einer Sekunde bis maximal fünf Minuten, besonders bevorzugt bis zu maximal 2,5 Minuten. Dies hat zur Folge, dass eine Gesamtmessdauer von weniger als zehn Minuten, vorzugsweise weniger als fünf Minuten erreichbar ist.

Vorzugsweise wird das Verfahren zur quantitativen Bestimmung von Legionellen verwendet. Es können jedoch auch andere Bakterien, insbesondere humanpathogene Bakterien wie Salmonellen, Listerien, coliforme Bakterien wie E. coli, z.B. EHEC, oder Krankenhauskeime wie MRSA bestimmt werden. Die Probe, in der die Bakterien bestimmt werden, kann eine Wisch- oder Wasserprobe, aber auch eine biologische Probe, z.B. eine Lebensmittelprobe aus Fleisch-, Milch- oder Eiprodukten, oder eine Körperflüssigkeit wie etwa Blut, Plasma, Serum, Urin etc. sein.

Die Bestimmung der Bakterien durch das erfindungsgemäße Verfahren erfolgt durch Verwendung eines mit den zu bestimmenden Bakterien bindefähigen Markierungsreagenz und Messung eines Signals, das von dem an die Bakterien gebundenen Markierungsreagenz stammt. Die Bestimmung kann beispielsweise durch Lumineszenzmessung, Fluoreszenzmessung und/oder durch Messung von Raman-Strahlung, z.B. mittels oberflächenverstärkter Raman-Spektroskopie (SERS) erfolgen. Dabei wird ein an die zu bestimmenden Bakterien bindefähiges Markierungsreagenz, vorzugsweise ein fluoreszentes, lumineszentes oder Raman-aktives Markierungsreagenz verwendet, das für die zu bestimmenden Bakterien selektiv ist, d.h. eine Struktur auf oder in den zu bestimmenden Bakterien erkennt, die nicht in anderen Bestandteilen der Probe vorkommt. Beispielsweise kann das Markierungsreagenz ausgewählt werden aus markierten Nukleinsäuresonden, Antikörpern, Bakteriophagen und Kombinationen davon. Bevorzugt ist das Markierungsreagenz ein markierter Bakteriophage.

In einer Ausführungsform kann ein fluoreszentes oder lumineszentes Markierungsreagenz verwendet werden, das Fluoreszenz- oder Lumineszenzstrahlung z.B. im Bereich von 200 nm bis 10 µm, insbesondere von 200 nm bis 2 µm emittiert. Besonders bevorzugte Emissionswellenlängen liegen im Bereich von 1300-1500 nm.

Fluoreszenz- oder lumineszenzmarkierte Nukleinsäuresonden, die zur Hybridisierung mit komplementären bakteriellen Nukleinsäuresequenzen verwendet werden, sind grundsätzlich bekannt und werden z.B. für die FISH-Technik verwendet. Die Nukleinsäuresonden sind üblicherweise Sonden auf Basis von Desoxyribonukleotid- und/oder Nukleinsäure-Analoga, z.B. LNA-Bausteinen. Sie können beispielsweise gegen ribosomale RNA- und/oder DNA-Sequenzen gerichtet sein, die für das nachzuweisende Bakterium spezifisch sind.

Auch markierte Antikörper, z.B. fluoreszenz- oder lumineszenzmarkierte Antikörper, d.h. polyklonale oder monoklonale Antikörper sowie Antikörperfragmente oder -derivate, die gegen ein für das nachzuweisende Bakterium spezifisches Antigen, z.B. ein auf der Bakterienoberfläche befindliches Antigen, gerichtet sind, sind bekannt.

In einer weiteren besonders bevorzugten Ausführungsform kann das Markierungsreagenz ein Bakteriophage sein, der eine Markierung, z.B. eine Fluoreszenz- oder Lumineszenzmarkierung trägt und spezifisch Oberflächenstrukturen auf dem zu bestimmenden Bakterium erkennt. Bakteriophagen für Legionellen sind z.B. bei Lammetyn (Microb. Ecol. 56 (2000), 191-197) beschrieben. Bakteriophagen für Salmonella sind z.B. bei Atterbury et al. (Appl. Environ. Microbiol. 73 (2007), 4543-4549) Wichard et al. (J. Food Prot 2 (2003), 178-340) oder deLappe et al. (J. Med. Microbiol. 58 (2009) beschrieben. Bakteriophagen von Listeria sind z.B. bei Klumpp & Loessner (www. Ncbi. Nlm.nih.gov>PMC3827098) beschrieben. Bakteriophagen für MRSA sind z.B. bei Sahin et al. (Mikrobiol. Bul. 47 (2013), 27-34) beschrieben. Bakteriophagen für E. coli, z.B. T-Phagen, Lambda-Phagen oder andere, sind bei Sambrook et al. (Molecular Cloning, A Laboratory Manual) beschrieben.

Für das erfindungsgemäße Verfahren werden markierte Bakteriophagen, z.B. fluoreszenz- oder lumineszenzmarkierte Bakteriophagen vorzugsweise in einem Überschuss bezüglich der nachzuweisenden Bakterien verwendet. Sie können etwa 10⁷-10⁹ Phagen pro Ansatz verwendet werden. Üblicherweise findet man, dass unter diesen Bedingungen jede Bakterienzelle mit etwa 50-400 Phagen, insbesondere mit etwa 50-250 oder 50-150 Phagen belegt wird. Eine E. coli Bakterienzelle kann beispielsweise mit etwa 70 Phagen belegt werden.

In einer weiteren bevorzugten Ausführungsform kann ein durch Raman-Spektroskopie nachweisbares Markierungsreagenz verwendet werden, z.B. ein Markierungsreagenz, das ein oberflächenverstärktes ("surface enhanced") Raman-spektroskopisch aktives Partikel enthält. Dieses Markierungsreagenz umfasst vorzugsweise ein metallisches Partikel, z.B. ein Gold- oder Silberpartikel und/oder ein Raman-Reportermolekül, das an eine an die nachzuweisenden Bakterien bindefähige Sonde gebunden ist. Das Raman-Reportermolekül kann z.B. aus einem Isothiocyanat- oder einem Multi-Schwefel-Fluoreszenzfarbstoff ausgewählt werden. Die Sonde kann wie zuvor beschrieben aus Nukleinsäuren, Antikörpern und Bakteriophagen, welche selektiv an die zu bestimmenden Bakterien binden, ausgewählt werden.

Die Fluoreszenz- oder Lumineszenzgruppen des Markierungsreagenz können aus beliebigen bekannten organischen oder anorganischen Fluoreszenz- oder Lumineszenzfarbstoffen ausgewählt werden, die an das Markierungsreagenz vorzugsweise kovalent gebunden sind. Geeignete Fluoreszenzgruppen sind Fluoreszenzfarbstoffe, beispielsweise Fluoresceine, Rhodamine, Oxazine oder Phycoerythrine, Fluoreszenzproteine wie GFP oder Varianten davon sowie fluoreszierende Quantendots. Geeignete Nachweisgruppen zur oberflächenverstärkten Raman-Spektroskopie (SERS) sind Substanzen, die eine oder mehrere Isothiocyanat-Gruppen und/oder Schwefelatome umfassen.

Das Inkubieren der nachzuweisenden Bakterien mit dem Markierungsreagenz erfolgt in einer Reaktionskammer. Hierzu wird die zu untersuchende Probe in die Reaktionskammer eingebracht. Das Markierungsreagenz kann der Probe vor oder nach dem Einbringen in die erste Kammer in Kontakt gebracht werden. Vorzugsweise enthält die erste Kammer ein Depot mit einer vorbestimmten Menge des Markierungsreagenz, das dort in flüssiger oder in trockener Form vorliegen kann. Vorzugsweise liegt das Markierungsreagenz in trockener und nach Kontakt mit der Probenflüssigkeit rekonstituierbarer Form vor. Die erste Kammer, in der die Inkubation der zu bestimmenden Bakterien mit dem Markierungsreagenz erfolgt, ist auf mindestens einer Seite durch eine Membran begrenzt sein. Alternativ kann die mit dem Markierungsreagenz versetzte Probe aus der Inkubationskammer in eine weitere Kammer geleitet werden, die mit einer Membran begrenzt ist. Diese Membran ist für die zu bestimmenden Bakterien undurchlässig, aber für das Markierungsreagenz, d.h. für ein nicht an Bakterien gebundenes Markierungsreagenz, durchlässig. Diese Membran weist beispielsweise einen Porendurchmesser von etwa 0,5-3 µm, z.B. 1-2 µm, auf. Geeignete Membranen, z.B. aus Kunststoffen wie Nylon, sind bekannt.

In einer bevorzugten Ausführungsform wird im erfindungsgemäßen Bestimmungsverfahren eine Messzelle verwendet, die mindestens zwei Kammern und eine zwischen den Kammern angeordnete Membran enthält, welche für die zu bestimmenden Bakterien undurchlässig und für nicht an die zu bestimmenden Bakterien gebundenes Markierungsreagenz durchlässig ist. Beispielsweise kann die Messzelle eine erste Kammer für die Inkubation und eine zweite Kammer zur Aufnahme abgetrennter Probenbestandteile und überschüssiger Markierung umfassen, die über eine Membran wie oben beschrieben in Verbindung stehen.

Nach Beendigung der Inkubation wird nicht an die Bakterien gebundenes Markierungsreagenz durch die Membran entfernt und die zu bestimmenden Bakterien auf der Membran gesammelt. Dieser Schritt kann durch Anlegen von Unterdruck an die Membran durchgeführt werden, so dass mit der Membran in Kontakt befindliche Probenflüssigkeit und nicht gebundenes Markierungsreagenz durch die Membran hindurch treten und die Bakterien auf der Membran gesammelt werden. Alternativ kann die Entfernung von Probenflüssigkeit und nicht gebundenem Markierungsreagenz auch durch Zentrifugation erfolgen. Vorzugsweise wird die von den Bakterien abgetrennte Probenflüssigkeit und das nicht gebundene Markierungsreagenz in eine hinter der Membran befindliche zweite Kammer geleitet.

Vor oder/und während der Inkubation kann eine homogene Durchmischung des Markierungsreagenz und der zu untersuchenden Probe erfolgen. Hierzu kann ein Vibrationselement vorgesehen werden, welches gegebenenfalls in der Messvorrichtung integriert ist. Nach der Inkubation können die markierten Bakterien gegebenenfalls ein- oder mehrmals gewaschen werden.

Die zur Inkubation vorgesehene erste Kammer ist so ausgestaltet, dass sie ein Probenvolumen aufnehmen kann, in dem sich eine für eine quantitative Bestimmung ausreichende Menge an Bakterien befindet. Üblicherweise ist die Reaktionskammer für ein Probenvolumen im Bereich von 0,5 ml oder größer, z.B. 1-100 ml, bevorzugt 5-50 ml, vorgesehen. Besonders bevorzugt ist ein Volumen im Bereich von etwa 10 ml.

Das erfindungsgemäße Verfahren beruht auf einer Bestimmung des an die gesammelten Bakterien gebundenen Markierungsreagenz durch Messung einer für die Markierungsgruppe charakteristischen Strahlung, z.B. von Fluoreszenz-, Lumineszenz- und/oder Raman-Strahlung. Hierzu wird Anregungslicht aus einer Lichtquelle auf die gesammelten Bakterien eingestrahlt und die von dem auf den Bakterien befindlichen Markierungsreagenz emittierte Fluoreszenz-, Lumineszenz- und/oder Raman-Strahlung gemessen. In einer bevorzugten Ausführungsform erfolgt die Messung, indem die gesammelten Bakterien nach Entfernen der Probenflüssigkeit und des überschüssigen Markierungsreagenz sowie gegebenenfalls einem oder mehreren Waschschritten direkt auf der Membran untersucht werden.

Als Lichtquelle wird vorzugsweise ein Laser, z.B. ein Quantenkaskadenlaser, verwendet. Dieser Laser steht in Verbindung mit einer geeigneten Optik, um eine Bestrahlung der gesammelten Bakterien, vorzugsweise eine flächige Bestrahlung der gesammelten Bakterien, vorzugsweise eine flächige Bestrahlung der auf der Membran gesammelten Bakterien, zu ermöglichen. Die Einkopplung des Lasersystems in die Reaktionskammer kann durch optisch transparente Fasern, z.B. Glasfasern, erfolgen.

Zum Nachweis von Fluoreszenz oder Lumineszenz kann als Laser eine Multimode- oder Singlemode-Laserdiode im UV-Bereich, z.B. mit einer Einstrahlwellenlänge von 350-420 nm, verwendet werden. Bevorzugt ist eine Einstrahlwellenlänge von 365 nm und 405 nm, die unter Verwendung von kommerziell verfügbaren Standardlaserdioden erzielt werden kann. Zum Nachweis von Raman-Strahlung kann auch ein Laser einer geeigneten Einstrahlwellenlänge im UV-, VIS- oder Nah-IR-Bereich verwendet werden.

Die Bestimmung des an die Bakterien gebundenen Markierungsreagenz kann durch Fluoreszenz-Spektroskopie, vorzugsweise durch Fluoreszenz-Korrelations-Spektroskopie und/oder durch Raman-Spektroskopie erfolgen. Dabei wird mittels einer geeigneten konfokalen Optik das von der Lichtquelle stammende Anregungslicht in die Probe fokussiert, um ein Anregungsvolumen zu erzeugen, in dem sich die zu bestimmenden Bakterien befinden.

Die von dem an die Bakterien gebundenen Markierungsreagenz emittierte Strahlung, z.B. Fluoreszenz-, Lumineszenz und/oder Raman-Strahlung, wird mit einem Photodetektor gemessen. Der Photodetektor kann z.B. eine Avalanche-Photodiode oder eine EMCCD Kamera sein. Vorzugsweise wird als Photodetektor ein Spektrometer eingesetzt, das eine spektrale Auflösung des emittierten Lichts über einen Bereich von mindestens 50 nm, mindestens 100 nm und bis zu 200 oder 250 nm ermöglicht. Vorzugsweise wird ein Photodetektor, z.B. ein Spektrometer mit einem Messbereich verwendet, der zwischen 200 bis 2000 nm liegt. Die optische Auflösung des Spektrometers beträgt 0,35-1 nm, vorzugsweise etwa 0,6-0,9 nm.

In einer Ausführungsform werden die markierten Bakterien durch oberflächenverstärkte Raman-Spektroskopie (SERS) bestimmt. Das Verfahren basiert auf der Anregung eines Raman-Reportermoleküls durch monochromatisches Laserlicht, wobei ein spezifisches Raman-Spektrum emittiert wird. Durch die Wechselwirkung des Raman-Reportermoleküls mit einem metallischen Nanopartikel, an das es gebunden ist, wird das Signal verstärkt. Bevorzugte metallische Nanopartikel bestehen aus einem Edelmetall wie Silber oder Gold und können unterschiedliche Strukturen und Größen aufweisen (Wang et al., Chem. Rev. 2013, 113, 1391-1428, EP 2 134 642 B1).

Das im Photodetektor erhaltene Messsignal wird quantitativ ausgewertet. Als Ergebnis erhält man die in der zu untersuchenden Probe befindliche Anzahl an Bakterien, die mit der gemessenen Fluoreszenz-, Lumineszenz- und/oder Raman-Strahlung korreliert. Die gemessene Anzahl an Bakterien entspricht einem bestimmten Wert koloniebildender Einheiten (KBE) pro Probenvolumen.

Die auf Basis des Messsignals bestimmte Anzahl an Bakterien kann anhand von Grenzwerten in eine oder mehrere Kategorien eingestuft werden. Diese Grenzwerte korrelieren mit einer für das jeweilige Verfahren spezifischen Fluoreszenzintensität. Beim Nachweis von Legionellen kann beispielsweise ein erster Grenzwert festgelegt werden, der einer Anzahl von 100 KBE pro 100 ml entspricht. Wenn die Anzahl der bestimmten Bakterien diesen Wert übersteigt, sind Maßnahmen zur Bekämpfung der Legionellen in dem entsprechenden Wassersystem zu veranlassen. Als weiterer Grenzwert kann eine Anzahl von 10.000 KBE pro 100 ml festgelegt werden. Wenn die Anzahl der bestimmten Bakterien diesen Wert übersteigt, sind Notfallmaßnahmen verbunden mit einer sofortigen Stilllegung des entsprechenden Wassersystems zu veranlassen.

Zur Unterstützung der quantitativen Auswertung kann eine Kalibrierung, z.B. durch die Verwendung von mindestens einem Referenzsignal, welches durch einen Referenzlaser erzeugt werden kann, durchgeführt werden. Alternativ kann eine Kalibrierung auch durch Verwendung interner Standards, z.B. fluoreszierender, lumineszierender oder Raman-aktiver Partikel, die eine von dem optisch nachweisbaren Markierungsreagenz unterschiedliche Fluoreszenz-, Lumineszenz- oder Ramanmarkierung tragen, erfolgen.

Ein weiterer Gegenstand der vorliegenden Erfindung ist eine Messzelle zur quantitativen Bestimmung von Bakterien, umfassend:
(i) eine erste Kammer, die gegebenenfalls ein Depot mit einem Markierungsreagenz für die zu bestimmenden Bakterien, z.B. Bakteriophagen, enthält, wobei das Markierungsreagenz vorzugsweise ausgewählt ist aus einem fluoreszenten Markierungsreagenz, einem lumineszenten Markierungsreagenz und einem Raman-Markierungsreagenz,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist.

Noch ein weiterer Gegenstand der vorliegenden Erfindung ist eine Vorrichtung zur quantitativen Bestimmung von Bakterien, umfassend:
(i) eine Messzelle wie zuvor angegeben,
(ii) Mittel zum Abziehen von überschüssigem Markierungsreagenz aus der ersten Kammer in die zweite Kammer,
(iii) Mittel zur Anregung der Strahlung, z.B. der Fluoreszenz-, Lumineszenz- und/oder Raman-Strahlung, von markierten, auf der Membran gesammelten Bakterien und
(iv) Mittel zur quantitativen Auswertung eines von den gesammelten Bakterien stammenden Messsignals.

Die erfindungsgemäße Messzelle und Vorrichtung können in einem Verfahren zur quantitativen Bestimmung von Bakterien, insbesondere in dem zuvor beschriebenen erfindungsgemäßen Verfahren, verwendet werden. Besonders bevorzugt ist die Verwendung zur quantitativen Bestimmung von Legionellen in einer Wasserprobe.

Die Erfindung soll weiterhin durch die folgenden Abbildungen erläutert werden:

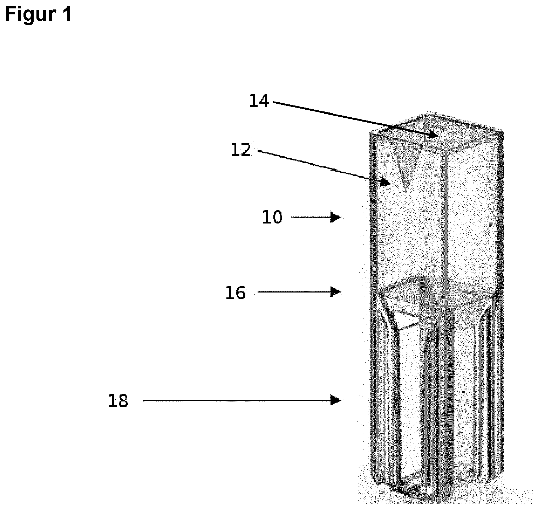

**Abbildung 1** zeigt eine Messzelle, die in Form einer Messküvette aufgebaut ist. Die Messzelle enthält eine Reaktionskammer oder erste Kammer (10) zur Aufnahme eines Probenvolumens von beispielsweise 10 ml. In der ersten Kammer (10) ist ein Markerdepot (12) mit einer vorbestimmten Menge an Markierungsreagenz, vorzugsweise in trockener Form, vorgesehen. Die erste Kammer (10) enthält auf einer Seite eine Öffnung (14) zur Aufnahme der Probe. Weiterhin steht die erste Kammer (10) über eine Membran (16) mit einer zweiten Kammer (18) in Verbindung. Die Membran (16) ist so ausgebildet, dass sie für in der ersten Kammer (10) befindliche Bakterien undurchlässig, aber für nicht an Bakterien gebundenes Markierungsreagenz durchlässig ist.

Zur Messung wird die in der ersten Kammer (10) befindliche Flüssigkeit nach Inkubation mit dem Markierungsreagenz in die zweiten Kammer (18) geleitet. In der Probe vorhandene Bakterien mit daran gebundenem Markierungsreagenz werden auf der Membran (16) abgelagert. Nicht gebundenes Markierungsreagenz kann die Membran (16) durchdringen und gelangt mit der Flüssigkeit in die zweite Kammer (18). Die Messung der auf der Membran (16) abgelagerten Bakterien kann ohne weitere Maßnahme *in situ* erfolgen.

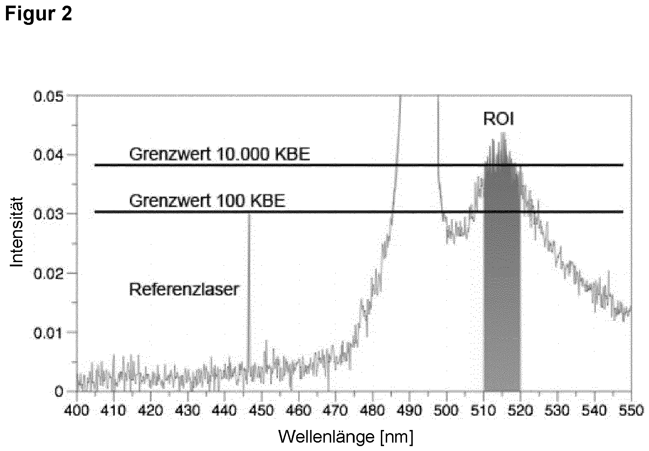

**Abbildung 2** zeigt das Ergebnis einer spektralen Messung an einer für die zu bestimmenden Bakterien, z.B. E. coli oder Legionellen, positiven Probe. Die Aufzeichnung des Messsignals erfolgt durch ein Spektrometer, dessen Messbereich vorzugsweise zwischen 350 und 550 nm liegt. Die optische Auflösung des Spektrometers liegt vorzugsweise bei 0,4-0,1 nm. Das von den zu bestimmenden Bakterien stammende Fluoreszenzsignal liegt im Bereich zwischen 510 und 520 nm (ROI). Durch einen Referenzlaser wird gewährleistet, dass die Amplitude des Spektrums einen korrekten Bezugswert hat. Die Auswertung umfasst die Festlegung von zwei Grenzwerten für Bakterienzahlen von 100 KBE pro 100 ml bzw. 10.000 KBE pro 100 ml. In der Abbildung übersteigt die Intensität des Messsignals den Grenzwert von 10.000 KBE. Folglich ist die bestimmte Probe extrem stark kontaminiert.

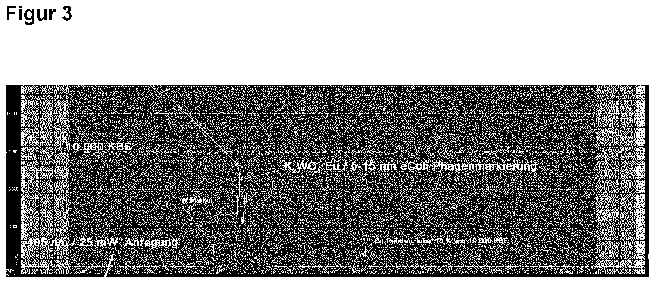

**Abbildung 3** zeigt das Ergebnis der Bestimmung einer Probe mit E. coli Bakterien. Als Markierungsreagenz wurden E. coli Phagen gekoppelt mit dem anorganischen Farbstoff K₂WO₄:Eu (Partikelgröße 5-15 nm) verwendet.

Die in der Probe enthaltenen Bakterien wurden nach Inkubation mit dem Markierungsreagenz in einer Reaktionskammer auf einer Membran abgelagert und dort bestimmt. Hierzu wurde die Membran mit einem Laser (405 nm/25 mW) homogen ausgeleuchtet und die bei einer Wellenlänge von 610-615 nm erzeugte Emissionsstrahlung quantitativ bestimmt. Die quantitative Umrechnung des in korrigierten Impulsen gemessenen Signals mit der Anzahl koloniebildender Einheiten (KBE) in der Probe war durch vorherige Eichung unter Verwendung von Standardproben mit einer bekannten Anzahl von E. coli Bakterien, z.B. im Bereich zwischen 10-10⁹ Zellen, möglich.

Im Spektrum ist außerdem der Peak eines Ca-Referenzlasers bei 702 nm zu erkennen, der mit einer Intensität entsprechend 10% des Grenzwerts von 10.000 KBE eingestrahlt wurde.

Im Bereich zwischen 590 und 600 nm ist noch ein - nicht zur quantitativen Bestimmung der Bakterien verwendetes - Signal des im Farbstoff enthaltenen Wolfram-Markers erkennbar.

Die Bestimmung der in Abbildung 3 gezeigten Probe ergab ein Signal, das einer Bakterienanzahl von 9.030 KBE entspricht. Durch Überprüfung mittels FACS konnte diese Bakterienanzahl verifiziert werden.

## Patentansprüche

1. Verfahren zur quantitativen Bestimmung von Bakterien in einer Probe umfassend die Schritte:
(a) Inkubieren der zu untersuchenden Probe mit einem an die zu bestimmenden Bakterien bindefähigen Markierungsreagenz, vorzugsweise ausgewählt aus einem fluoreszierenden Markierungsreagenz, einem lumineszierenden Markierungsreagenz und einem Raman-Markierungsreagenz,
(b) Entfernen von nicht an die Bakterien gebundenem Markierungsreagenz von den Bakterien durch eine für die zu bestimmenden Bakterien undurchlässige und für das Markierungsreagenz durchlässige Membran aus der, wobei die zu bestimmenden Bakterien auf der Membran gesammelt werden,
(c) Bestimmen des an die gesammelten Bakterien gebundenen Markierungsreagenz vorzugsweise durch Fluoreszenz-, Lumineszenz- und/oder Ramanmessung und
(d) quantitatives Auswerten des Messsignals aus Schritt (c).

2. Verfahren nach Anspruch 1,
**dadurch gekennzeichnet,**
**dass** die Bakterien ausgewählt werden aus Legionellen, Salmonellen, Listerien, coliformen Keimen wie E. coli, Krankenhauskeimen wie MRSA.

3. Verfahren nach Anspruch 1 oder 2,
**dadurch gekennzeichnet,**
**dass** das Markierungsreagenz ausgewählt wird aus markierten Nukleinsäuresonden, Antikörpern, Bakteriophagen und Kombinationen davon.

4. Verfahren nach einem der Ansprüche 1 bis 3,
**dadurch gekennzeichnet,**
**dass** man als Markierungsreagenz einen markierten Bakteriophagen, vorzugsweise fluoreszenz-, lumineszenz- oder Raman-markierten Bakteriophagen verwendet.

5. Verfahren nach einem der Ansprüche 1 bis 4,
**dadurch gekennzeichnet,**
**dass** zur Bestimmung eine Messzelle verwendet wird, umfassend
(i) eine erste Kammer, die gegebenenfalls ein Depot für das Markierungsreagenz enthält,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist, und
wobei nach Beendigung des Inkubationsschrittes nicht an die zu bestimmenden Bakterien gebundenes Markierungsreagenz aus der ersten Kammer entfernt und durch die Membran in die zweite Kammer geleitet wird.

6. Verfahren nach einem der Ansprüche 1 bis 5,
**dadurch gekennzeichnet,**
**dass** eine Membran mit einem Porendurchmesser von etwa 0,5-3 µm verwendet wird.

7. Verfahren nach einem der Ansprüche 1 bis 6,
**dadurch gekennzeichnet,**
**dass** vor und/oder während der Inkubation gemäß Schritt (a) eine Durchmischung des Markierungsreagenz und der zu untersuchenden Probe erfolgt.

8. Verfahren nach einem der Ansprüche 1 bis 7,
**dadurch gekennzeichnet,**
**dass** für die optische Bestimmung gemäß Schritt (c) ein Laser mit einer konfokalen Optik als Lichtquelle verwendet wird.

9. Verfahren nach Anspruch 8,
**dadurch gekennzeichnet,**
**dass** der Laser in einer integrierten Vorrichtung mit einem Spektrometer als Detektionseinheit verwendet wird.

10. Verfahren nach einem der Ansprüche 1 bis 9,
**dadurch gekennzeichnet,**
**dass** die quantitative Auswertung gemäß Schritt (d) die Einstufung der bestimmten Anzahl von Bakterien in eine oder mehrere Kategorien anhand von vorbestimmten Grenzwerten umfasst.

11. Verfahren nach einem der Ansprüche 1 bis 10,
**dadurch gekennzeichnet,**
**dass** für die quantitative Auswertung gemäß Schritt (d) mindestens ein Referenzsignal, insbesondere ein durch einen Laser erzeugtes Referenzsignal, verwendet wird.

12. Messzelle zur quantitativen Bestimmung von Bakterien, umfassend:
(i) eine erste Kammer, die gegebenenfalls ein Depot für ein Markierungsreagenz enthält, wobei das Markierungsreagenz vorzugsweise ausgewählt ist aus einem fluoreszenten Markierungsreagenz, einem lumineszenten Markierungsreagenz und einem Raman-Markierungsreagenz,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist.

13. Messzelle nach Anspruch 12,
**dadurch gekennzeichnet,**
**dass** das Volumen der ersten Kammer 2-20 ml beträgt.

14. Messzelle nach Anspruch 12 oder 13,
**dadurch gekennzeichnet,**
**dass** das Markierungsreagenz ein markierter Bakteriophage ist.

15. Vorrichtung zur quantitativen Bestimmung von Bakterien, umfassend:
(i) eine Messzelle nach einem der Ansprüche 12 bis 14,
(ii) Mittel zum Abziehen von überschüssigem Markierungsreagenz aus der ersten Kammer in die zweite Kammer,
(iii) Mittel zur Anregung von Strahlung, vorzugsweise von Fluoreszenz-, Lumineszenz- und/oder Raman-Strahlung von markierten, auf der Membran gesammelten Bakterien und
(iv) Mittel zur quantitativen Auswertung eines von den gesammelten Bakterien stammenden Messsignals.

16. Verwendung der Messzelle nach einem der Ansprüche 12 bis 14 oder der Vorrichtung nach Anspruch 15 in einem Verfahren zur quantitativen Bestimmung von Bakterien, insbesondere in einem Verfahren nach einem der Ansprüche 1 bis 11.

17. Verwendung nach Anspruch 16 zur quantitativen Bestimmung von Legionellen in einer Wasserprobe.
